(19) Europäisches Patentamt
European Patent Office
Office européen des brevets

(11) **EP 3 394 613 B1**

(12) **FASCICULE DE BREVET EUROPEEN**

(45) Date de publication et mention
de la délivrance du brevet:
**09.10.2019 Bulletin 2019/41**

(21) Numéro de dépôt: **16826332.5**

(22) Date de dépôt: **22.12.2016**

(51) Int Cl.:
***G01N 33/487*** (2006.01)

(86) Numéro de dépôt international:
**PCT/EP2016/082290**

(87) Numéro de publication internationale:
**WO 2017/109024 (29.06.2017 Gazette 2017/26)**

(54) **PROCÉDÉ POUR PRÉDIRE LA BIODISPONIBILITÉ D'UN RADIOÉLÉMENT SUITE À UNE CONTAMINATION ET SES UTILISATIONS**

VERFAHREN ZUR VORHERSAGE DER BIOVERFÜGBARKEIT EINES RADIOELEMENTS NACH DER KONTAMINATION UND VERWENDUNGEN DAVON

METHOD FOR PREDICTING THE BIOAVAILABILITY OF A RADIOELEMENT FOLLOWING CONTAMINATION, AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.12.2015 FR 1563214**

(43) Date de publication de la demande:
**31.10.2018 Bulletin 2018/44**

(73) Titulaires:
• **Commissariat à l'énergie atomique et aux énergies alternatives 75015 Paris (FR)**
• **Orano Cycle 92400 Courbevoie (FR)**

(72) Inventeurs:
• **GRIFFITHS, Nina 91190 Gif Sur Yvette (FR)**
• **VAN DER MEEREN, Anne 77166 Evry Gregy Sur Yerres (FR)**

(74) Mandataire: **Brevalex 56, Boulevard de l'Embouchure B.P. 27519 31075 Toulouse Cedex 2 (FR)**

(56) Documents cités:
**US-A- 3 983 007**

• SARKAR H S ET AL: "Syntheses of Several <99m>Tc and <131>I Labeled Neoglycoalbumins and Their Differential Uptake Patterns in Animal Biodistribution Experiments", NUCLEAR MEDICINE AND BIOLOGY, ELSEVIER, NY, US, vol. 22, no. 5, 1 juillet 1995 (1995-07-01), pages 589-597, XP004051756, ISSN: 0969-8051, DOI: 10.1016/0969-8051(95)00003-G
• J. C. RENSHAW ET AL: "Development and Application of an Assay for Uranyl Complexation by Fungal Metabolites, Including Siderophores", APPLIED AND ENVIRONMENTAL MICROBIOLOGY, vol. 69, no. 6, 1 juin 2003 (2003-06-01), pages 3600-3606, XP055292991, US ISSN: 0099-2240, DOI: 10.1128/AEM.69.6.3600-3606.2003
• GRIFFITHS N M ET AL: "Forecasting the In Vivo Behavior of Radiocontaminants of Unknown Physicochemical Properties Using a Simple In Vitro Test", HEALTH PHYSICS FEB 2010 LNKD-PUBMED:20065682,, vol. 111, no. 2, 1 août 2016 (2016-08-01), pages 93-99, XP008181078, ISSN: 1538-5159

**Description**

**DOMAINE TECHNIQUE**

La présente invention concerne le domaine général de la contamination notamment humaine par les radioéléments.

Plus particulièrement, la présente invention propose un procédé pour prédire la biodisponibilité des radioéléments tels que les actinides suite à une contamination. Ce procédé met en oeuvre un compartiment statique mimant la zone de contamination qui correspond également à la zone de rétention du radioélément et un compartiment dynamique mimant le fluide biologique en contact avec la zone de contamination et dans lequel le radioélément peut se dissoudre et être entraîné en vue de son élimination.

La présente invention concerne également l'utilisation d'un tel procédé pour identifier une molécule apte à chélater les radioéléments et/ou pour caractériser les propriétés chélatantes d'une telle molécule.

**ÉTAT DE LA TECHNIQUE ANTÉRIEURE**

La contamination humaine par des radioéléments tels que des actinides peut intervenir dans les activités industrielles liées au cycle du combustible nucléaire : extraction minière de l'uranium, purification chimique et conversion de l'uranium, séparation isotopique, fabrication de combustibles nucléaires, traitement du combustible nucléaire irradié et recyclage, assainissement et démantèlement d'installations nucléaires, transports de matières nucléaires, ou encore entreposage et stockage de matières ou déchets nucléaires. Elle peut également intervenir dans des laboratoires de recherche travaillant dans un quelconque domaine lié au nucléaire ou impliquant l'utilisation de radioéléments. Cette contamination peut survenir par inhalation, par ingestion, par blessure et/ou par contact.

Suite à une telle contamination, il est nécessaire de pouvoir apprécier le comportement des radioéléments dans l'organisme et ce, pour évaluer les moyens disponibles pour limiter leurs effets sur l'organisme et leur dangerosité. Pour cela, il faut tenir compte de leur solubilité : un composé soluble sera éliminé rapidement par l'organisme, alors qu'un composé peu ou pas soluble restera plus longtemps dans l'organisme.

A cet effet, il existe différentes méthodes de dissolution *in vitro* utilisées pour les actinides et décrites par Ansoborlo *et al,* 1999 **[1]**. Schématiquement, ces méthodes sont basées sur le dépôt du composé à tester sur une membrane. La fraction solubilisée est récupérée par filtration d'un tampon au travers de la membrane suite à l'incubation de la membrane en présence de ce tampon. Une autre technique est basée sur l'incubation de particules dans un tampon, suivie d'une centrifugation pour séparer les particules de la fraction dissoute. Un autre système emprisonne des particules entre deux membranes, et la fraction solubilisée est récupérée dans le compartiment extérieur du système. Toutefois, aucune de ces techniques ne s'avère suffisamment fiable ou reproductible.

On peut encore citer la technique basée sur le principe de la diffusion des espèces métalliques dissoutes à travers un gel de diffusion bien défini et leur accumulation sur une membrane échangeuse d'ions. Cette technique initialement décrite par Davison et Zhang, 1994 **[2]** est appelée technique en gradient de diffusion simple ou technique DGT pour « Diffusive Gradient in Thin film ». Cette dernière est notamment employée pour étudier les métaux labiles dans les eaux, les sédiments et les sols.

De plus, sont décrits, dans la littérature, des essais de dissolution de particules métalliques, principalement sous forme nanométrique, dans des milieux mimant des fluides biologiques. Toutefois, ces tests sont réalisés en conditions statiques, donc très éloignées des conditions rencontrées dans un organisme vivant et reposent essentiellement sur une séparation particules/élément dissous.

Dans le domaine pharmaceutique clairement distinct de celui de la contamination par des radioéléments, des hydrogels comme des gels d'agarose sont déjà utilisés pour étudier la diffusion d'une drogue. Ainsi, Klose *et al,* 2009 **[3]** décrivent une méthode d'évaluation *in vitro* de diffusion d'une drogue pharmaceutique. Cette méthode est basée sur une étude de la diffusion d'un composé pharmaceutique au sein d'un gel d'agarose à 0,1%-0.6%. Plus particulièrement, une fois le gel d'agarose solidifié, un trou est percé au centre du gel, constituant un réservoir dans lequel est ajoutée une solution comprenant le composé à caractériser. Des échantillons du gel sont prélevés à différentes distances du réservoir et la quantité de composé dans les échantillons prélevés est évaluée. Hoang Thi *et al,* 2010 **[4]** reprennent ce principe de l'utilisation de gels d'agarose pour étudier la diffusion d'un composé pharmaceutique. Chaibva et Walker, 2007 **[5]** comparent, quant à eux, différentes méthodes d'évaluation de libération *in vitro* d'une drogue pharmaceutique, les gels utilisés étant des gels d'agarose à 20-30%. Sont également connues et utilisées, dans le domaine pharmaceutique, des compositions permettant de mimer un compartiment biologique donné **[6]**.

Dans le domaine de la physiologie, clairement distinct de celui de la contamination par des radioéléments, des mesures de la capacité absorptive dans le côlon, principalement le mouvement d'eau et d'électrolytes (Na+, K+), ont été réalisés *in vivo*. L'avantage de l'agarose est de permettre la diffusion fluidique et ionique **[7]**.

Enfin, il convient de souligner que la manipulation des radioéléments et notamment des actinides comme le plutonium (Pu) soulève des difficultés d'ordre technique. En effet, du fait de la très forte activité massique des isotopes du Pu (>$10^9$ Bq/g), il est quasi impossible, à partir d'une suspension particulaire de procéder à un échantillonnage reproductible en terme d'activité. La reproductibilité inter échantillons est meilleure pour les composés plus solubles. De plus, en raison de la forte adsorption du Pu sur toute surface et principalement sur les plastiques, l'échantillonnage doit être réalisé sur une très courte période de temps pour être reproductible. Dans ce contexte H. S. Sarkar et al. (Nucl. Med. Biol. 1995) décrit un procédé pour prédire la disponibilité d'un radioélément dans un animal en injectent le radioélément dans l'animal.

Ainsi, les tests mettant en oeuvre des radioéléments comme des actinides doivent être réalisés très rapidement après la préparation des échantillons, ce qui peut engendrer des incertitudes sur leur fiabilité. En d'autres termes, en raison de l'activité massique des isotopes et de leur forte adsorption sur les supports, il est très difficile de réaliser des tests fiables et reproductibles impliquant des radioéléments comme les actinides.

Il existe donc un réel besoin pour un procédé *in vitro* pour prédire ou déterminer la biodisponibilité des radioéléments *in vivo* suite à une contamination humaine, tenant compte des spécificités et contraintes propres aux radioéléments et ne présentant pas les inconvénients des procédés connus de l'art antérieur.

## EXPOSÉ DE L'INVENTION

Le procédé selon l'invention satisfait ce besoin et présente en outre d'autres avantages. En effet, les inventeurs ont mis au point un procédé permettant de prédire le comportement et donc la biodisponibilité *in vivo* d'un radioélément suite à une contamination d'un être vivant par ce dernier, ledit procédé étant rapide, relativement simple de mise en oeuvre, ne nécessitant pas d'instrumentation particulière, sauf pour la mesure du radioélément libéré. A noter que ce procédé est acellulaire i.e. il ne nécessite l'utilisation d'aucune cellule ou culture cellulaire pour sa mise en oeuvre.

En effet, les inventeurs proposent un procédé impliquant deux éléments essentiels qui sont (1) un gel mimant la zone de contamination (compartiment statique) dans lequel se trouve le radioélément dont on souhaite déterminer la biodisponibilité et (2) un liquide en contact avec le gel et dans lequel le radioélément est susceptible d'être libéré, un tel liquide soumis à une agitation mimant le compartiment dynamique en contact avec la zone contaminée.

Comme précédemment expliqué, le procédé selon l'invention permet d'évaluer la capacité d'un radioélément à quitter la zone de contamination. Si le radioélément étudié et incorporé dans le gel ne passe pas dans le compartiment dynamique, alors cela signifie qu'il reste dans la zone de contamination et donc qu'il est insoluble et/ou piégé au niveau de cette dernière et, par conséquent, potentiellement dangereux.

Le fait de préparer, pour la mise en oeuvre du procédé, une solution de gel unique d'une composition donnée contenant le radioélément à étudier puis de la fractionner permet de garantir une très grande reproductibilité de la « quantité » notamment en termes de masse et d'activité du radioélément étudié, y compris s'il est sous forme particulaire. De plus, cette préparation est utilisable aussi bien pour des composés solubles, modérément solubles ou encore insolubles.

Le procédé selon l'invention permet de changer facilement la composition du compartiment statique et/ou du compartiment dynamique et donc non seulement de tester de très nombreuses compositions mimant différentes conditions biologiques mais aussi d'évaluer l'influence de nombreux paramètres sur la dissolution/biodisponibilité d'un radioélément à tester comme la masse, la composition des compartiments de rétention et le pH. Ces propriétés associées au fait que le procédé selon l'invention offre la possibilité de traiter de très nombreux échantillons dans les mêmes conditions et en même temps (plusieurs centaines) permettent qu'un tel procédé se définisse comme une technique haut débit.

Enfin, le procédé selon l'invention met en oeuvre des volumes de gel (compartiment statique) ou de liquide en contact avec ce gel (compartiment dynamique) relativement faibles typiquement et respectivement inférieurs à 1 ml et à 5 ml permettant d'obtenir un test prédictif miniature.

Plus particulièrement, la présente invention concerne un procédé pour prédire la biodisponibilité d'un radioélément dans un organisme vivant animal suite à une contamination de cet organisme par ledit radioélément, comprenant les étapes consistant à :

a) préparer un gel dans lequel ledit radioélément est uniformément réparti, ledit gel mimant la zone de contamination dans ledit organisme vivant animal ;
b) mettre le gel préparé à ladite étape (a) en contact avec une solution mimant un fluide biologique associé à la zone de contamination dans ledit organisme vivant animal puis placer l'ensemble sous agitation et
c) mesurer, à un instant t, la quantité dudit radioélément dans ladite solution, ladite mesure permettant de prédire la biodisponibilité dudit radioélément dans ledit organisme vivant animal.

A noter que les expressions « fluide biologique » et « liquide biologique » sont utilisées indistinctement dans la présente description.

Le terme « biodisponibilité » désigne, dans le cadre de la présente invention, la proportion de radioéléments qui agit effectivement, à l'instant t de mesure, dans l'organisme vivant par rapport à la quantité totale de radioéléments auxquels l'organisme est exposé au moment de la contamination. *In vitro,* cette biodisponibilité peut être exprimée en proportion de radioélément libéré dans la solution à l'instant t à partir du gel préparé à l'étape (a) par rapport à la quantité totale de radioélément initialement placée dans ce gel lors de sa préparation. Par conséquent, la biodisponibilité du radioélément à l'instant t se calcule selon la formule suivante (I) :

$$\text{Biodisponibilité (à t)} = (\text{quantité dans la solution à t})/(\text{quantité totale initiale}) \times 100 \quad (I)$$

Par « organisme vivant », on entend tout organisme vivant animal et notamment un être humain.

La présente invention s'applique à tout radioélément naturel ou artificiel, susceptible de contaminer un organisme vivant. Il est précisé que les termes « radioélément » et « radionucléide » sont utilisés indistinctement dans la présente description. Dans un mode de réalisation particulier, un tel radioélément est un actinide. A titre d'exemples plus particuliers d'actinides dont on peut souhaiter prédire la biodisponibilité, on peut citer l'uranium, le plutonium, le thorium, l'américium, le curium, le neptunium ou un quelconque de leur mélange. Dans le cadre de la présente invention, le radioélément étudié peut se présenter sous différentes formes chimiques, notamment d'un oxyde, d'un nitrate, d'un fluorure, ou métallique, et/ou sous forme solide, soluble ou non.

Comme précédemment indiqué, le procédé selon l'invention met en oeuvre un gel. Les gels sont généralement formés à partir d'au moins deux constituants : une solution (ci-après désignée solution du gel) qui est un liquide « piégé » par un second composé qui forme un filet à trois dimensions, ou réseau tridimensionnel, dans toute la solution. Ce réseau tridimensionnel est constitué de composés à propriétés colloïdales susceptibles de former une matrice i.e. une phase continue solide. Typiquement, un gel est un matériau mou, gonflé de solution, capable de subir de grandes déformations.

Dans le cadre de la présente invention, tous les composés à propriétés colloïdales généralement employés pour la préparation des gels, notamment en biologie, sont utilisables dans le cadre de l'invention. Avantageusement, ces composés à propriétés colloïdales sont de nature organique : il s'agit généralement de macromolécules qui, typiquement, sont des molécules de masse moléculaire relative élevée dont la structure est formée essentiellement d'unités à multiples répétitions dérivées, de fait ou d'un point de vue conceptuel, de molécules de masse moléculaire faible.

Ainsi, par exemple, il est possible d'utiliser, comme composés à propriétés colloïdales, des macromolécules comme les polysaccharides et, notamment, l'agarose, le sucrose, le sépharose, le chitosan, le xanthane, le carraghénane, le dextran, l'agar, l'alginate ou un de leurs mélanges. Il est en effet possible d'employer des mélanges de deux polysaccharides différents tels qu'un mélange de carraghénane et d'agarose voire plus de deux. On peut également citer à titre d'exemple un gel de polyacrylamide ou d'un dérivé de ce dernier qui est formé par polymérisation d'acrylamide ou d'un dérivé d'acrylamide en présence d'un agent de réticulation.

Avantageusement, lors de la préparation du gel mis en oeuvre dans le cadre du procédé selon l'invention, le rapport composé(s) à propriétés colloïdales /solution du gel (poids exprimé en g/volume exprimé en ml) est compris entre 0,01 et 0,05, typiquement entre 0,02 et 0,04. Il est par exemple possible de préparer un gel d'agarose dans lequel le rapport agarose/solution du gel est de 2,5% i.e. de 0,25 g d'agarose / 10 ml de solution du gel.

Le gel mis en oeuvre dans le cadre de la présente invention présente la propriété de mimer la zone de contamination par le radioélément. La notion de « mimer » signifie que le gel est un modèle représentatif de la zone contaminée ou d'un des éléments (tissus ou cellules) présents au niveau de la zone contaminée et dans lesquels le ou les radioélément(s) peu(ven)t être retenu(s). Ce modèle présente des propriétés physicochimiques notamment en terme de pH et de concentrations ionique, protéique et lipidique comparables ou analogues à celles de la zone contaminée ou de l'élément et présente éventuellement un ou plusieurs composé(s) constitutif(s) caractéristique(s) de la zone contaminée ou dudit élément, le gel se distinguant toutefois de la zone contaminée par l'absence de cellules. La solution du gel et/ou des composés additionnels retenus dans le réseau tridimensionnel du gel permettent de conférer, à ce dernier, ses propriétés mimétiques.

Par conséquent, la solution du gel utilisée dans le procédé selon l'invention est typiquement une solution aqueuse comme, par exemple, du sérum physiologique composé d'eau distillée et de chlorure de sodium (NaCl) dilué à une concentration de 8 à 9 g.L$^{-1}$, éventuellement complété(e) par un ou plusieurs sels et notamment un ou plusieurs sels organique(s) ou inorganique(s). Ces sels et notamment ces sels organiques ou inorganiques sont utilisés pour modifier la concentration ionique et le pH de la solution. A titre d'exemples de tels sels organiques ou inorganiques, on peut citer le chlorure de sodium (NaCl), le chlorure de potassium (KCl), le bicarbonate de sodium (NaHCO$_3$), le phosphate de sodium (Na$_3$PO$_4$), l'hydrogénophosphate de sodium (Na$_2$HPO$_4$), le dihydrogénophosphate de sodium (NaH$_2$PO$_4$), l'hydrogénophosphate de potassium (K$_2$HPO$_4$), le dihydrogénophosphate de potassium (KH$_2$PO$_4$), l'acide chlorhydrique (HCl), le chlorure de magnésium (MgCl$_2$), le chlorure de calcium (CaCl$_2$), le sulfate de sodium (Na$_2$SO$_4$), l'hydroxyde de sodium (NaOH), l'hydroxyde de potassium (KOH), l'acétate de sodium (CH$_3$COONa), le tartrate de sodium

($Na_2C_4H_4O_6$), le lactate de sodium ($C(OH)(CH_3)COONa$), le pyruvate de sodium ($CH_3C(O)COONa$), le citrate de sodium ($Na_3C_6H_5O_7$) et l'acide citrique ($C_6H_8O_7$).

La zone contaminée mimée peut être un quelconque élément ou compartiment d'un organisme humain susceptible d'être contaminé suite à l'inhalation ou l'ingestion d'un radioélément ou suite à un contact ou une blessure en présence de ce dernier. Ainsi, la zone contaminée peut être la muqueuse pulmonaire, la muqueuse buccale, la muqueuse gastrique, la cornée, le derme, le foie (suite au processus d'élimination d'un radioélément contaminant), un muscle, du cartilage, de l'os, de la synovie (ou liquide synovial), le suc gastrique, le mucus ou surfactant pulmonaire et un phagolysosome (ou vacuole de phagocytose) présent(e) dans les cellules phagocytaires.

Il est clair que pour étudier la biodisponibilité d'un radioélément suite à une contamination, par exemple par blessure, il est possible de mettre en oeuvre le procédé selon l'invention en utilisant plusieurs gels mimant chacun un élément ou compartiment : muscle, cartilage, os, synovie (ou liquide synovial), tous susceptibles d'être contaminés lors de cette blessure mais aussi phagolysosome suite au recrutement de macrophages circulant vers la zone blessée.

Comme précédemment expliqué, le gel peut présenter un ou plusieurs composé(s) constitutif(s) caractéristique(s) de la zone contaminée mimée ou d'un des éléments présents au niveau de la zone contaminée mimée. Ce ou ces composé(s) appartiennent, pour la majorité, aux familles des glycoprotéines et des glycosaminoglycanes. Ce ou ces composé(s) est/sont notamment choisi(s) dans le groupe constitué par la pepsine, la lécithine, la glycine, du glucose, le lysozyme, l'albumine, le taurocholate de sodium, l'acide maléique, la transferrine, la ferritine, la fibrine, l'acide hyaluronique, la glucosamine, la fibronectine, la laminine, une mucine, les kératines, les collagènes, la chondroïtine, l'ostéopontine, l'hydroxyapatite et l'acide glutamique. L'homme du métier au vu de ses connaissances saura choisir le ou les composé(s) adéquat(s) et la quantité à mettre en oeuvre dans le gel en fonction de la zone de contamination mimée. Il pourra notamment se rapporter aux gels fournis dans la partie expérimentale ci-après et mimant le liquide synovial, le cartilage, une matrice extracellulaire simple (MEC1) ou plus complexe (MEC2). Le MEC2 inclut le collagène mais également d'autres composés tels que la glucosamine (un « building block » de tout monosaccharide) et l'acide hyaluronique, un glycosaminoglycan étant le composé majeur de cette matrice. Ces deux gels ne représentent pas de tissus distincts.

L'étape (a) du procédé selon la présente invention consiste à préparer un gel dans lequel le radioélément à étudier est uniformément réparti. Par « uniformément réparti », on entend que la quantité en radioélément dans deux volumes identiques de gel choisis indépendamment l'un de l'autre sera sensiblement identique. La notion de répartition uniforme s'oppose au cas où le radioélément est accumulé dans un site donné du gel tel qu'un évidement formant un réservoir de radioéléments.

L'étape de préparation du gel est fonction des composés employés et est connue dans le domaine correspondant. Elle est généralement effectuée par la mise en solution des composés à propriétés colloïdales puis par la formation du gel. Le gel peut se former spontanément. S'agissant des composés chimiques, tels que les polysaccharides, il est généralement nécessaire de procéder à un chauffage puis à un refroidissement de la solution dans laquelle se trouvent les composés à propriétés colloïdales. Le chauffage assure la dispersion des composés dans la solution et permet de rompre une partie des liaisons faibles existant entre les différents composés qui peuvent alors se réorganiser pour former un réseau tridimensionnel. Le gel apparaît lors du refroidissement de la solution. Par exemple, pour ce qui est des gels de polyacrylamide, la formation du gel nécessite l'utilisation d'un agent de réticulation et d'acrylamide.

Avantageusement, le gel dans lequel le radioélément est uniformément réparti peut être préparé en une étape unique. Pour cela, il est souhaitable d'ajouter directement le radioélément lors de la préparation du gel. Le procédé comportera alors une étape de préparation d'un gel à partir d'au moins un composé à propriétés colloïdales, d'une solution telle que précédemment définie (solution du gel) et d'au moins un radioélément.

Comme précédemment explicité, la présente invention s'applique aussi bien à des radioéléments solubles qu'à des radioéléments peu ou pas solubles. Ainsi, la mise en solution d'un radioélément solide aboutit à une solution (dissolution complète d'un radioélément soluble) ou à une suspension (radioélément peu ou pas soluble).

Dans un mode de réalisation particulier, le radioélément sous forme de solution est repris dans une solution du gel telle que précédemment définie à laquelle est ajouté au moins un composé à propriétés colloïdales. Ce mode de réalisation s'applique typiquement aux radioéléments solubles ou moyennement solubles. Par exemple, une activité (en Bq) connue d'un radioélément est placée dans une fiole en verre. Après évaporation sur plaque chauffante, le radioélément est repris dans une solution qui sera ajoutée à une solution du gel. Cette préparation permet de disposer d'une solution de concentration précise qui servira de référence, l'activité de la solution et le volume de reprise étant contrôlés.

En variante, le radioélément est ajouté sous forme d'une suspension à la solution du gel contenant au moins un composé à propriétés colloïdales. Cette variante s'applique notamment aux radioéléments insolubles ou peu solubles comme les oxydes métalliques. Typiquement, une suspension du radioélément est préparée en boîte à gants et le volume de suspension contenant l'activité désirée est ajouté directement dans la solution du gel contenant déjà au moins un composé à propriétés colloïdales telle qu'une solution d'agarose. Dans cette variante, le volume de suspension ajouté à la solution du gel est inférieur ou égal à 1% du volume de la solution du gel.

Lors de la préparation du gel, il est bien entendu possible d'ajouter un ou plusieurs composé(s) constitutif(s) caractéristique(s) tels que précédemment définis. Ce ou ces derniers peu(ven)t être ajouté(s) à la solution du gel avant, après ou pendant l'ajout du radioélément et/ou à la solution du gel avant, après ou pendant l'ajout du composé à propriétés colloïdales.

Il est avantageux de donner au gel une morphologie choisie, cette dernière étant souvent élaborée lors de la préparation dudit gel. Il est, pour cela, possible d'employer des moules particuliers correspondant à la forme que l'utilisateur souhaite donner au gel. Il est également possible de modeler, après sa préparation, le gel obtenu et ce, à l'aide d'outils adaptés tels que des lames. Typiquement, dans le cadre de la présente invention, la composition comprenant la solution du gel, au moins un composé à propriétés colloïdales, le radioélément et éventuellement au moins un composé constitutif caractéristique est versée, préalablement à toute solidification, dans un puits d'une plaque multi-puits classiquement utilisée en biologie ou en biotechnologie. A titre d'exemple, un volume compris entre 500 et 700 $\mu$l de composition est ajouté dans un puits tel qu'un puits de 3,9 $cm^2$ d'une plaque 12 puits.

Une fois le gel comprenant le radioélément uniformément réparti préparé conformément à l'étape (a) du procédé selon la présente invention, il est possible de mettre en oeuvre l'étape (b) immédiatement après ou, au contraire, de façon retardée. En effet, avant cette mise en oeuvre, il est possible de conserver le gel préparé à l'étape (a) dans un emballage hermétique pour éviter le dessèchement et les changements de propriétés du gel. Typiquement, cette conservation est réalisée à 4°C pendant une durée n'excédant pas 36 h et notamment n'excédant pas 24 h.

L'étape (b) du procédé selon la présente invention consiste à mettre le gel contenant le radioélément et mimant la zone de contamination en contact avec une solution mimant un fluide biologique associé à ladite zone de contamination dans ledit organisme vivant.

Par « fluide biologique associé à la zone de contamination », on entend un fluide biologique avec lequel la zone de contamination telle que précédemment définie entretient *in vivo* des échanges tels que des échanges liquides, des échanges de nutriments et/ou des échanges de déchets. Ce fluide biologique peut être *in vivo* en contact fluidique continu avec la zone de contamination i.e. irriguer la zone de contamination. En variante, ce fluide biologique est un fluide circulant, s'écoulant ou coulant *in vivo* à proximité de la zone de contamination. A titre d'exemples, de tels liquides biologiques, on peut citer le sang, la lymphe, la salive, les larmes, l'humeur aqueuse de la chambre antérieure (au niveau de l'oeil), le liquide interstitiel et la sueur. De plus, pour une zone de contamination donnée, plusieurs liquides biologiques différents peuvent être associés à cette dernière. Par exemple, pour une contamination par ingestion, le fluide biologique associé à la muqueuse buccale et via lequel le contaminant peut être éliminé peut être soit le sang irriguant cette muqueuse, soit la salive.

L'étape (b) du procédé selon l'invention met en oeuvre une solution mimant un tel liquide biologique. Tout ce qui a été précédemment défini pour le gel mimant la zone de contamination s'applique *mutatis mutandis* à la solution mimant le fluide biologique associé à la zone de contamination. Ainsi, cette solution présente des propriétés physicochimiques notamment en terme de pH, de viscosité et de concentrations ionique, protéique et lipidique, comparables ou analogues à celles du liquide biologique mimé et peut éventuellement comprendre un ou plusieurs composé(s) constitutif(s) caractéristique(s) de ce liquide, la solution se distinguant toutefois de ce dernier par l'absence de cellules.

Avantageusement, la solution de l'étape (b) est une solution aqueuse comprenant un ou plusieurs sel(s) organique(s) ou inorganique(s) choisi(s) dans le groupe constitué par le chlorure de sodium (NaCl), le chlorure de potassium (KCl), le bicarbonate de sodium ($NaHCO_3$), le phosphate de sodium ($Na_3PO_4$), l'hydrogénophosphate de sodium ($Na_2HPO_4$), le dihydrogénophosphate de sodium ($NaH_2PO_4$), l'hydrogénophosphate de potassium ($K_2HPO_4$), le dihydrogénophosphate de potassium ($KH_2PO_4$), l'acide chlorhydrique (HCl), le chlorure de magnésium ($MgCl_2$), le chlorure de calcium ($CaCl_2$), le sulfate de sodium ($Na_2SO_4$), l'hydroxyde de sodium (NaOH), l'hydroxyde de potassium (KOH), l'acétate de sodium ($CH_3COONa$), le tartrate de sodium ($Na_2C_4H_4O_6$), le lactate de sodium ($C(OH)(CH_3)COONa$), le pyruvate de sodium ($CH_3C(O)COONa$), le citrate de sodium ($Na_3C_6H_5O_7$) et l'acide citrique ($C_6H_8O_7$).

A titre d'exemples de composés constitutifs susceptibles d'être plus particulièrement ajoutés dans la solution de l'étape (b), on peut citer la pepsine, la lécithine, la glycine, du glucose, le lysozyme, l'albumine, le taurocholate de sodium, l'acide maléique, la transferrine, la ferritine et la fibrine.

L'homme du métier connaît différentes solutions utilisées pour mimer un fluide biologique d'un organisme vivant. Ainsi, Marques *et al,* 2011 **[6]** décrivent la composition de plusieurs de ces solutions utilisables dans le cadre de la présente invention. A titre d'exemples particuliers, un milieu de culture (type RPMI 1640 ou MEM) additionné de 10% de sérum de veau foetal et la solution de Gambles peuvent être utilisés pour mimer, respectivement, du sang et du fluide pulmonaire.

A noter que, selon le gel et la solution associés l'un à l'autre, la composition de la solution du gel et celle de la solution mise en oeuvre à l'étape (b) peuvent être soit identiques soit différentes. Ainsi, la solution du gel et la solution mise en oeuvre à l'étape (b) comprennent un ou plusieurs sels, identiques ou différents.

La mise en contact entre la solution et le gel lors de l'étape (b) du procédé peut être réalisée par toute technique classique permettant de disposer un gel dans une solution ou d'appliquer une solution sur un gel. Ainsi, lorsque le gel est préparé dans un puits d'une plaque multi-puits, la solution mimant le liquide biologique est répartie sur le gel au moyen d'une pipette et ce, de façon à ce que la solution recouvre le gel. A titre d'exemple, un volume compris entre 2 et 3 ml de solution est ajouté dans un puits tel qu'un puits de 3,9 cm$^2$ d'une plaque 12 puits au fond duquel se trouve le gel.

De plus, lors de l'étape (b) et afin de mimer les conditions existant *in vivo* entre la zone de contamination et le fluide biologique associé à cette dernière et notamment le mode dynamique de cette mise en contact, le gel mimant la zone de contamination et la solution mimant ce fluide biologique sont soumis à une agitation. Ainsi, le terme « l'ensemble » précédemment utilisé pour l'étape (b) correspond au gel mis en contact avec la solution. Avantageusement, cette agitation se fait à une vitesse relativement lente. Par « vitesse relativement lente », on entend, dans le cadre de la présente invention, une vitesse de rotation inférieure ou égale à 250 tr/min, notamment supérieure ou égale à 2 tr/min et, en particulier, comprise entre 5 et 150 tr/min. Plus particulièrement, la plaque multi-puits dont au moins un des puits présente un gel recouvert d'une solution, tel que précédemment défini, est disposée sur un agitateur présentant une vitesse de rotation de 5 tr/min, et placée dans un incubateur.

De même, la mise en contact et l'agitation lors de l'étape (b) sont réalisées dans des conditions proches de celles rencontrées dans un organisme vivant et ce, notamment en terme de température et d'atmosphère environnante. Avantageusement, l'étape (b) est effectuée dans des incubateurs classiquement utilisés dans le domaine de la culture cellulaire i.e. comme un incubateur agitateur présentant une température de l'ordre de 37°C (i.e. 37°C $\pm$ 5°C et notamment 37°C $\pm$ 3°C) et dont la composition contrôlée de l'atmosphère est de l'air humide comprenant 5% de $CO_2$. Il est également possible de réaliser l'étape (b) dans des conditions stériles, permettant d'étudier le comportement du radioélément réparti dans le gel dans des solutions riches i.e. propices aux contaminations bactériennes ou fongiques sur des temps longs.

En fonction du radioélément étudié dans le procédé selon l'invention, l'homme du métier saura déterminer, sans effort inventif, la ou lestechnique(s) utilisable(s) pour mesurer, à un instant t, la quantité de radioélément dans la solution en contact avec le gel ce qui correspond à l'étape (c) du procédé selon la présente invention. Le choix sera notamment fonction du type de rayonnement émis par le radioélément. A titre d'exemples illustratifs de techniques utilisables pour cette mesure, on peut citer les techniques notamment spectrométriques utilisant des détecteurs à gaz, des détecteurs à semiconducteur tel que le germanium, le tellure de cadmium et le silicium, des détecteurs à scintillation inorganique utilisant comme scintillateur l'iodure de sodium, l'iodure de césium ou le germanate de bismuth, des détecteurs à scintillation organique liquide ou encore des détecteurs à scintillation organique solide.

De même, les éventuelles conditions de traitement de la solution avant cette étape de mesure dépendent non seulement du radioélément étudié mais aussi de la technique de mesure sélectionnée. L'homme du métier saura choisir, sans effort inventif, celles qui seront le mieux adaptées au radioélément et à la technique de mesure. Ceci s'applique également aux éventuelles conditions de traitement du gel notamment en vue de déterminer la quantité totale de radioélément mise en oeuvre comme exposé dans la partie expérimentale ci-après.

A noter que le temps t de mesure s'apprécie par rapport au to qui se définit comme l'instant à partir duquel la solution et le gel sont mis en contact lors de l'étape (b).

La présente invention concerne également l'utilisation d'un procédé tel que précédemment défini pour identifier une molécule présentant des propriétés chélatantes vis-à-vis d'un radioélément donné et/ou pour caractériser les propriétés chélatantes d'une molécule.

La notion de « propriétés chélatantes » s'entend, dans le cadre de la présente invention, comme la capacité d'une molécule, naturelle ou synthétique, à se lier et à former un complexe stable avec un radioélément tel qu'un actinide. La formation d'un tel complexe permet de piéger le radioélément et d'empêcher les interactions non souhaitées en bloquant la réactivité normale du radioélément. De telles molécules sont appelées « agents chélatants » mais aussi « agents décorporants » du fait de leur capacité à entraîner la décorporation du radioélément i.e. l'excrétion hors de l'organisme vivant du radioélément par les voies naturelles (urinaire et fécale).

Le procédé de la présente invention offre plusieurs avantages et spécificités particulièrement adaptés à l'identification et à la caractérisation de molécules chélatantes comme, par exemple, la possibilité de tester de très nombreuses compositions mimant différentes zones de contamination ou de fluides biologiques associés à ces dernières. De plus, le fait que ce procédé puisse être mis en oeuvre avec des volumes de gel ou de solution en contact avec ce dernier relativement faibles permet de ne nécessiter que de faibles quantités de molécules à tester.

Ainsi, la présente invention concerne un procédé pour identifier une molécule présentant des propriétés chélatantes vis-à-vis d'un radioélément comprenant les étapes consistant à

i) mesurer la biodisponibilité dudit radioélément selon un procédé tel que précédemment défini dans lequel la solution mimant le fluide biologique comprend une molécule susceptible de présenter des propriétés chélatantes,

ii) mesurer la biodisponibilité dudit radioélément selon un procédé tel que précédemment défini dans les mêmes conditions que dans l'étape (i), la solution mimant le fluide biologique étant dépourvue de ladite molécule susceptible de présenter des propriétés chélatantes et

iii) comparer la biodisponibilité obtenue à l'étape (i) et celle obtenue à l'étape (ii) moyennant quoi une biodisponibilité obtenue à l'étape (i) supérieure à celle obtenue à l'étape (ii) est une indication que la molécule testée à l'étape (i) présente des propriétés chélatantes vis-à-vis dudit radioélément.

Dans un tel procédé, on utilise, lors des étapes (i) et (ii), les mêmes conditions en termes de composition du gel mimant une zone de contamination, de nature et quantité de radioélément initialement présent dans ce gel, de composition de la solution mimant le fluide biologique, de technique de mesure du radioélément présent dans la solution et de temps t à laquelle cette mesure est réalisée, la seule différence étant la présence ou l'absence de la molécule à tester dans la solution mimant le fluide biologique. De plus, il est possible d'obtenir un effet dose-réponse en réalisant l'étape (i) en présence de différentes quantités de la molécule à tester.

Avec des stratégies comparables, il est possible :

- de comparer les propriétés chélatantes d'une molécule donnée vis-à-vis de différents radioéléments et ce, par mise en oeuvre d'un procédé selon l'invention en utilisant des gels de composition identique préparés de façon à contenir chacun une quantité identique d'un radioélément donné ;
- de comparer au moins deux molécules présentant des propriétés chélatantes vis-à-vis d'un même radioélément, la comparaison de la biodisponibilité du radioélément obtenue pour chaque molécule permettant de classer les propriétés chélatantes de ces dernières les unes par rapport aux autres ;
- de comparer les propriétés chélatantes d'une molécule donnée vis-à-vis d'un radioélément donné en fonction de la zone contaminée par ce radioélément et ce, en comparant la biodisponibilité du radioélément obtenue par mise en oeuvre de gels de compositions différentes ;
- de comparer les propriétés chélatantes d'une molécule donnée vis-à-vis d'un radioélément donné en fonction du fluide biologique par lequel elle atteint la zone contaminée par ce radioélément et ce, en comparant la biodisponibilité du radioélément obtenue par mise en oeuvre de solutions de compositions différentes en contact avec un gel de composition identique ; et
- de tester différentes concentrations de molécules chélatantes.

D'autres caractéristiques et avantages de la présente invention apparaîtront encore à l'homme du métier à la lecture des exemples ci-dessous donnés à titre illustratif et non limitatif et faisant référence aux figures annexées.

**BRÈVE DESCRIPTION DES DESSINS**

La Figure 1 présente la quantité de Bleu d'Evans récupérée par mise en oeuvre d'un procédé selon l'invention réalisé lors de différentes manipulations effectuées par deux opérateurs différents.

La Figure 2 présente l'activité $\alpha$ totale issue de MOX, de Pu en sels de nitrate et d'Am en sels de nitrate récupérée par mise en oeuvre d'un procédé selon l'invention.

La Figure 3 présente l'activité $\alpha$ totale de Pu récupérée à partir de gels mimant différents sites de contamination lors d'une blessure par mise en oeuvre d'un procédé selon l'invention.

La Figure 4 présente l'activité $\alpha$ totale de Pu récupérée par mise en oeuvre d'un procédé selon l'invention à partir de gels en contact avec différents milieux contenant ou non des molécules chélatantes (DTPA et 3,4-LIHOPO).

**EXPOSÉ DÉTAILLÉ DE MODES DE RÉALISATION PARTICULIERS**

**I. Répétatibilité et reproductibilité du procédé selon l'invention.**

1.1. Protocole opératoire.

Deux cent cinquante mg d'agarose (Type IIA ; Medium EEO) sont ajoutés à 10 ml de sérum physiologique (8,8 g/L NaCl, 0,37 g/L KCl) dans un bêcher. Ce mélange est pesé puis chauffé dans un four à micro-ondes (170 W x 30 s x 2 ; 170 W x 20 s x 1). Après 2 min de refroidissement, le bécher est de nouveau pesé afin d'ajuster le volume jusqu'à la valeur du volume initial avec du sérum physiologique.

Cent $\mu$l de bleu d'Evans (0,2% dans l'eau) sont alors ajoutés à l'agarose en mixant doucement. Après refroidissement d'environ 5-10 minutes, 700 $\mu$l de la solution d'agarose contenant le bleu d'Evans sont répartis dans les puits de culture (plaque de 12 puits) avec une pipette Gilson P1000 avec un cône dont la pointe a été coupée afin de faciliter le pipetage.

Trois ml de sérum physiologique sont ajoutés sur les gels. Les plaques sont incubées à 37°C en atmosphère humide et en présence de 5% de $CO_2$ pendant 2 h sous agitation lente (5 tr/min). Deux heures après le début de l'incubation, le milieu est prélevé dans chaque puits et le taux de bleu d'Evans est mesuré par spectrophotométrie à 610 nm. Les gels sont dissous dans du formamide et la densité optique est mesurée. Les concentrations de bleu d'Evans dans les échantillons sont calculées à partir d'une gamme de concentrations croissantes du colorant.

I.2. Résultats.

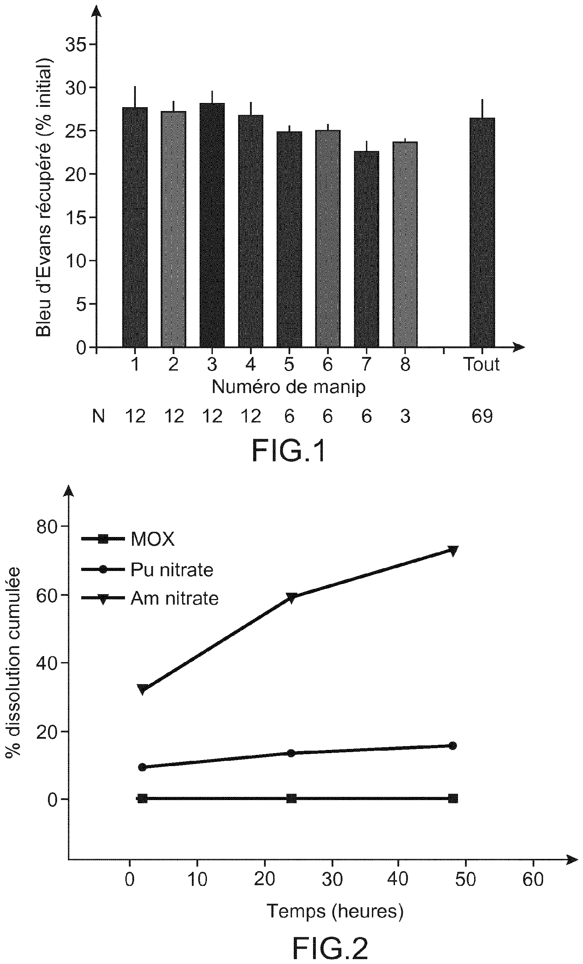

Huit expériences ont été réalisées dans des mêmes conditions sur plusieurs jours, et par deux opérateurs, avec 3 à 12 échantillons par expérience (Figure 1). Les données obtenues montrent une répétabilité pour un même opérateur avec un coefficient de variation d'environ 8,6% et une reproductibilité entre deux opérateurs avec un coefficient de variation d'environ 8,5%.

**II. Dissolution d'actinides.**

11.1. Protocole opératoire.

Les solutions et suspensions d'actinides sont préparées comme suit :

**MOX:** 25 mg de MOX (pour « Mixed OXide » i.e. mélange d'oxydes d'uranium et de plutonium) sont placés dans 5 ml d'éthanol absolu et vortéxés vigoureusement. Cinq cents $\mu$l de cette suspension sont dilués au ¼ dans du NaCl 0,9%. Un aliquot est prélevé pour mesure de l'activité $\alpha$ totale par scintillation liquide.

**Pu** : Un aliquot d'une solution de plutonium (Pu) en acide nitrique 2N (composition isotopique en % d'activité totale : 12,4% $^{239}$Pu, 85,6% $^{238}$Pu et 1,8% $^{241}$Am) d'environ 4-5 kBq est placé dans une fiole à scintillation liquide en verre, placée sur plaque chauffante (100°C) jusqu'à évaporation complète. Le Pu est alors repris dans 100 $\mu$l d'eau ou dans un tampon NaCl/KCl. L'activité totale est mesurée en scintillation liquide.

**Am** : Un aliquot d'une solution d'américium (Am) en acide nitrique 2N d'environ 4-5 kBq est placé dans une fiole à scintillation liquide en verre, placée sur plaque chauffante (100°C) jusqu'à évaporation complète. L'Am est alors repris dans 100 $\mu$l d'eau. L'activité totale est mesurée en scintillation liquide.

Une solution d'agarose à 2,5% est préparée dans du sérum physiologique (8,8 g/L NaCl, 0,37 g/L KCl, pH 5,6) par ajout de 0,375 g d'agarose (low melting point, A11-EE0 Sigma Aldrich) dans 15 ml de sérum physiologique dans un bêcher. La solution est chauffée pendant 30 s dans un four à micro-ondes à la puissance de 250 W, homogénéisée puis chauffée 30 s, de nouveau homogénéisée puis chauffée 20 s. Après avoir vérifié que la solution est bien translucide, le volume est réajusté à 15 ml si besoin.

Après refroidissement à température ambiante de 5 à 10 min, approximativement 100 kBq de MOX, 4-5 kBq de Pu ou d'Am dans 100-150 $\mu$l sont ajoutés dans les 15 ml de solution d'agarose. Le mélange est homogénéisé précautionneusement. 700 $\mu$l du mélange sont alors ajoutés dans des puits de 3,9 cm$^2$ de plaques de culture 12 puits, à l'aide d'une pipette automatique P1000 munie d'un cône dont le bout a été coupé. La plaque de culture est agitée très légèrement afin de répartir la solution d'agarose de façon homogène au fond du puits. La plaque est alors conservée 10-15 min à température ambiante, sans son couvercle pour éviter la condensation, pour atteindre une solidification complète des gels.

Chaque condition est réalisée *a minima* trois fois (3 puits identiques).

A cette étape, la plaque peut être soudée dans un vinyle étanche et conservée à +4°C pour une utilisation ultérieure (1-3 jours) ou utilisée immédiatement.

Trois ml de sérum physiologique sont alors ajoutés dans chaque puits. La plaque de culture est ensuite placée dans un incubateur de culture cellulaire à 37°C, en atmosphère humide et 5% de CO2, sous agitation lente (5 tr/min).

Deux heures après le début de l'incubation, le tampon de chaque puits est prélevé et placé dans une fiole à scintillation liquide. Quinze ml de scintillant (Ultimagold) sont alors ajoutés et l'activité $\alpha$ totale est mesurée par scintillation liquide (compteur Packard). Trois ml de tampon sont ajoutés sur chacun des puits, et la plaque est replacée dans l'incubateur. Un prélèvement des surnageants est réalisé de façon identique à 24 h et 48 h après le début de l'incubation.

A 48 h, les gels sont délicatement récupérés des plaques de culture à l'aide d'une spatule métallique et peuvent être placés dans une boîte de Pétri, autour de laquelle est placé un film du type Parafilm M® pour éviter le dessèchement pour comptage direct à l'aide d'un détecteur. En variante, les gels délicatement récupérés sont directement placés dans des fioles à scintillation liquide. 2 ml d'acide nitrique (2N) sont ajoutés dans les fioles. Celles-ci sont placées sur une plaque chauffante (100°C) jusqu'à évaporation complète. Un ml d'$H_2O_2$ (30%) est alors ajouté. Après évaporation complète, 1 ml d'acide nitrique 2N est ajouté, puis 15 ml de liquide scintillant. L'activité de chaque échantillon i.e. les surnageants déjà prélevés et le gel est alors mesurée.

II.2. Résultats.

Les activités de chaque puits mesurées dans les surnageants à 2 h, 24 h et 48 h sont ajoutées à celle des gels. Cette valeur constitue le 100% de chaque puits, c'est-à-dire l'activité initiale déposée dans les puits. Les résultats sont alors exprimés en activité mesurée dans les surnageants/activité initiale et présentés sur la Figure 2. A noter que l'activité à 24 h est égale à la somme de l'activité du surnageant à 2 h et du surnageant à 24 h et que l'activité à 48 h est la somme de l'activité du surnageant à 2 h, du surnageant à 24 h et du surnageant à 48 h.

Rapportés à l'activité initiale, il y a moins d'activité issue du MOX présent dans le surnageant que du Pu ou d'Am en sels de nitrate. Ces derniers sont donc moins retenus dans le gel d'agarose que le MOX : le Pu et l'Am sont, par conséquent, plus biodisponibles quand ils sont sous forme de sels de nitrate que contenus dans le MOX.

**III. Identification des compartiments de rétention du plutonium.**

III.1. Protocole opératoire.

La solution de Pu est préparée de façon identique à ce qui est décrit au point II.1 ci-dessus puis ajoutée dans la solution d'agarose préparée dans des tampons de composition variables, en fonction du compartiment physiologique mimé. Une solution d'agarose préparée dans du NaCl/KCl est utilisée comme contrôle.

**Pour mimer le liquide synovial (SYN)** : une solution 8,8 g/L NaCl, 0,37 g/L KCl, 1,44 g/L $Na_2HPO_4$, 0,24 g/L $KH_2PO_4$, 3 g/L d'acide hyaluronique est utilisée pour préparer le gel d'agarose.

**Pour mimer la matrice extracellulaire (MEC-1)** : une solution de 8 g/L NaCl, 0,2 g/L KCl, 1,44 g/L $Na_2HPO_4$, 0,24 g/L $KH_2PO_4$, est utilisée pour préparer le gel d'agarose comme décrit au point II.1 ci-dessus. Après refroidissement à température ambiante de 5 à 10 min, du collagène (type I, issu de queue de rat) est ajouté pour obtenir une concentration finale de 0,5 g/L. Le pH est ajusté à 7 avec de l'acide acétique.

**Pour mimer la matrice extracellulaire (MEC-2)** : une solution de 8 g/L NaCl, 0,2 g/L KCl, 1,44 g/L $Na_2HPO_4$, 0,24 g/L $KH_2PO_4$, 3 g/L acide hyaluronique, 1,1 g/L glucosamine est utilisée pour préparer le gel d'agarose. Après refroidissement à température ambiante de 5 à 10 min, du collagène (type I, issu de queue de rat) est ajouté pour obtenir une concentration finale de 0,5 g/L. Le pH est ajusté à 7 avec de l'acide acétique.

**Pour mimer le cartilage (CHON)** : une solution de 8 g/L NaCl, 0,2 g/L KCl, 1 g/L chondroïtine est utilisée pour préparer le gel d'agarose.

Après homogénéisation 4-5 kBq de Pu dans 100-150 $\mu$l sont ajoutés dans chacune des différentes solutions d'agarose. La suite de l'expérimentation est similaire à celle décrite au point II.1 ci-dessus.

III.2. Résultats.

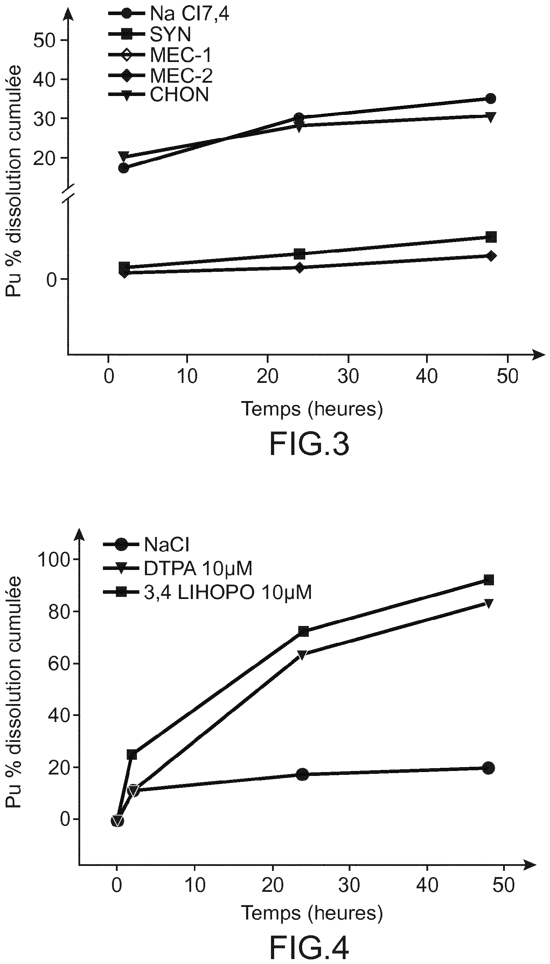

Comme exposé dans le protocole du point II ci-dessus, les activités de chaque puits mesurées dans les surnageants à 2 h, 24 h et 48 h sont ajoutées à celle des gels. Cette valeur constitue le 100% de chaque puits, c'est-à-dire l'activité initiale déposée dans les puits. Les résultats sont alors exprimés en activité mesurée dans les surnageants/activité initiale et présentés à la Figure 3.

Il ressort des compartiments biologiques mimés, deux types de comportement avec (1) les compartiments dans lesquels le Pu est retenu à savoir le liquide synovial et la matrice extracellulaire, les deux courbes correspondant à MEC-1 et MEC-2 se juxtaposant et (2) ceux qui ne retiennent pas plus le Pu que l'agarose contrôle à savoir le cartilage. De fait, le Pu apparaît moins biodisponible dans le liquide synovial et la matrice extracellulaire que dans le cartilage.

**IV. Evaluation de l'efficacité de molécules chélatantes du plutonium.**

IV.1. Protocole opératoire.

La solution de Pu et la solution d'agarose sont préparées de façon identique à ce qui est décrit au point II.1 ci-dessus. La préparation des gels contenant le Pu est réalisée comme décrit au point II.1 ci-dessus.

L'incubation des gels d'agarose se fait dans des tampons de compositions différentes en fonction de la molécule chélatante à tester. Une incubation dans un tampon NaCl/KCl est utilisée en contrôle. Une solution d'acide diéthylène triamine penta acétique (Ca-DTPA, Pharmacie centrale des armées) est diluée dans du NaCl/KCl pour obtenir une concentration à 10 $\mu$M. Une solution de 3,4-LIHOPO est préparée à concentration finale de 10 $\mu$M. La suite de l'expérimentation est similaire à celle décrite au point II.1 ci-dessus.

IV.2. Résultats.

Comme exposé dans le protocole du point II ci-dessus, les activités de chaque puits mesurées dans les surnageants à 2 h, 24 h et 48 h sont ajoutées à celle des gels. Cette valeur constitue le 100% de chaque puits, c'est-à-dire l'activité initiale déposée dans les puits. Les résultats sont alors exprimés en activité mesurée dans les surnageants/activité initiale et présentés à la Figure 4.

Comme attendu du fait de la nature de Ca-DTPA et de 3,4-LIHOPO, le gel d'agarose retient moins le Pu qui devient donc plus biodisponible lorsque le milieu entourant ce gel comprend une molécule chélatante comparé à un milieu n'en comprenant aucune.

**RÉFÉRENCES**

**[1]** Ansoborlo et al, 1999, « Review and critical analysis of available in vitro dissolution tests », Health Physics, vol. 77, pages 638-645.

**[2]** Davison and Zhang, 1994, « In situ speciation measurements of trace components in natural waters using thin film gels », Nature, vol. 367, pages 546-548.

**[3]** Klose et al, 2009, « Towards more realistic in vitro release measurement techniques for biodegradable microparticles », Pharmaceutical Reseach, vol. 26, pages 691-699.

**[4]** Hoang Thi et al, 2010, « Bone implants modified with cyclodextrin: Study of drug release in bulk », International Journal of Pharmaceutics, vol. 400, pages 74-85.

**[5]** Chaibva and Walker, 2007, « The comparison of in vitro release methods for the evaluation of oxytocin release from pluronic® F127 parenteral formulations », Dissolution Technologies, vol. 14, pages 15-25.

**[6]** Marques et al, 2011, « Simulated biological fluids with possible application in dissolution testing », Dissolution Technologies, vol. 18, pages 15-28.

**[7]** Zammit et al, 1994, « Effects on fluid and Na+ flux of varying luminal hydraulic restance in rat colon in vivo », Journal of Physiology, vol. 477, pages 539-548.

**Revendications**

1. Procédé pour prédire la biodisponibilité d'un radioélément dans un organisme vivant animal suite à une contamination de cet organisme par ledit radioélément, comprenant les étapes consistant à :

   a) préparer un gel dans lequel ledit radioélément est uniformément réparti, ledit gel mimant la zone de contamination dans ledit organisme vivant animal ;
   b) mettre le gel préparé à ladite étape (a) en contact avec une solution mimant un fluide biologique associé à la zone de contamination dans ledit organisme vivant animal puis placer l'ensemble sous agitation et
   c) mesurer, à un instant t, la quantité dudit radioélément dans ladite solution, ladite mesure permettant de prédire la biodisponibilité dudit radioélément dans ledit organisme vivant animal.

2. Procédé selon la revendication 1, **caractérisé en ce que** ledit radioélément est un actinide.

3. Procédé selon la revendication 1 ou 2, **caractérisé en ce que** le gel est préparé lors de ladite étape (a) à partir d'au moins un composé à propriétés colloïdales, d'une solution, désignée solution du gel, et d'au moins un radioélément.

4. Procédé selon la revendication 3, **caractérisé en ce que** ledit composé à propriétés colloïdales est un polysaccharide notamment choisi dans le groupe constitué par l'agarose, le sucrose, le sépharose, le chitosan, le xanthane, le carraghénane, le dextran, l'agar, l'alginate ou leurs mélanges.

**5.** Procédé selon la revendication 3 ou 4, **caractérisé en ce que** le rapport composé(s) à propriétés colloïdales (poids exprimé en g) /solution du gel (volume exprimé en ml) est compris entre 1 et 5%, typiquement entre 2 et 4%.

**6.** Procédé selon l'une quelconque des revendications 3 à 5, **caractérisé en ce que** ladite solution du gel est une solution aqueuse comme, par exemple, du sérum physiologique, éventuellement complété(e) par un ou plusieurs sels organiques ou inorganiques choisis dans le groupe constitué par le chlorure de sodium (NaCl), le chlorure de potassium (KCl), le bicarbonate de sodium (NaHCO$_3$), le phosphate de sodium (Na$_3$PO$_4$), l'hydrogénophosphate de sodium (Na$_2$HPO$_4$), le dihydrogénophosphate de sodium (NaH$_2$PO$_4$), l'hydrogénophosphate de potassium (K$_2$HPO$_4$), le dihydrogénophosphate de potassium (KH$_2$PO$_4$), l'acide chlorhydrique (HCl), le chlorure de magnésium (MgCl$_2$), le chlorure de calcium (CaCl$_2$), le sulfate de sodium (Na$_2$SO$_4$), l'hydroxyde de sodium (NaOH), l'hydroxyde de potassium (KOH), l'acétate de sodium (CH$_3$COONa), le tartrate de sodium (Na$_2$C$_4$H$_4$O$_6$), le lactate de sodium (C(OH)(CH$_3$)COONa), le pyruvate de sodium (CH$_3$C(O)COONa), le citrate de sodium (Na$_3$C$_6$H$_5$O$_7$) et l'acide citrique (C$_6$H$_8$O$_7$).

**7.** Procédé selon l'une quelconque des revendications précédentes, **caractérisé en ce que** le gel comprend un ou plusieurs composé(s) choisi(s) dans le groupe constitué par la pepsine, la lécithine, la glycine, du glucose, le lysozyme, l'albumine, le taurocholate de sodium, l'acide maléique, la transferrine, la ferritine, la fibrine, l'acide hyaluronique, la glucosamine, la fibronectine, la laminine, une mucine, les kératines, les collagènes, la chondroïtine, l'ostéopontine, l'hydroxyapatite et l'acide glutamique.

**8.** Procédé selon l'une quelconque des revendications 3 à 7, **caractérisé en ce que**, lors de ladite étape (a), le radioélément est ajouté sous forme d'une suspension ou d'une solution à la solution du gel contenant au moins un composé à propriétés colloïdales.

**9.** Procédé selon l'une quelconque des revendications précédentes, **caractérisé en ce que** la solution de ladite étape (b) est une solution aqueuse comprenant un ou plusieurs sels organiques ou inorganiques choisis dans le groupe constitué par le chlorure de sodium (NaCl), le chlorure de potassium (KCl), le bicarbonate de sodium (NaHCO$_3$), le phosphate de sodium (Na$_3$PO$_4$), l'hydrogénophosphate de sodium (Na$_2$HPO$_4$), le dihydrogénophosphate de sodium (NaH$_2$PO$_4$), l'hydrogénophosphate de potassium (K$_2$HPO$_4$), le dihydrogénophosphate de potassium (KH$_2$PO$_4$), l'acide chlorhydrique (HCl), le chlorure de magnésium (MgCl$_2$), le chlorure de calcium (CaCl$_2$), le sulfate de sodium (Na$_2$SO$_4$), l'hydroxyde de sodium (NaOH), l'hydroxyde de potassium (KOH), l'acétate de sodium (CH$_3$COONa), le tartrate de sodium (Na$_2$C$_4$H$_4$O$_6$), le lactate de sodium (C(OH)(CH$_3$)COONa), le pyruvate de sodium (CH$_3$C(O)COONa), le citrate de sodium (Na$_3$C$_6$H$_5$O$_7$) et l'acide citrique (C$_6$H$_8$O$_7$).

**10.** Procédé selon l'une quelconque des revendications 3 à 9, **caractérisé en ce que** ladite solution du gel et ladite solution mise en oeuvre à l'étape (b) comprennent un ou plusieurs sels, identiques ou différents

**11.** Procédé selon l'une quelconque des revendications précédentes, **caractérisé en ce que** la solution de ladite étape (b) comprend en outre un ou plusieurs composés choisis dans le groupe constitué par la pepsine, la lécithine, la glycine, du glucose, le lysozyme, l'albumine, le taurocholate de sodium, l'acide maléique, la transferrine, la ferritine et la fibrine.

**12.** Procédé selon l'une quelconque des revendications précédentes, **caractérisé en ce que** la solution de ladite étape (b) est effectuée dans un incubateur agitateur présentant une température de l'ordre de 37°C et dont la composition contrôlée de l'atmosphère est de l'air humide comprenant 5% de CO$_2$.

**13.** Utilisation d'un procédé selon l'une quelconque des revendications 1 à 12, pour identifier une molécule présentant des propriétés chélatantes vis-à-vis d'un radioélément donné et/ou pour caractériser les propriétés chélatantes d'une molécule.

**14.** Procédé pour identifier une molécule présentant des propriétés chélatantes vis-à-vis d'un radioélément comprenant les étapes consistant à :

i) mesurer la biodisponibilité dudit radioélément selon le procédé selon l'une quelconque des revendications 1 à 12 dans lequel la solution mimant le fluide biologique comprend une molécule susceptible de présenter des propriétés chélatantes,
ii) mesurer la biodisponibilité dudit radioélément selon le procédé selon l'une quelconque des revendications 1 à 12 dans les mêmes conditions que dans l'étape (i), la solution mimant le fluide biologique étant dépourvue de ladite molécule susceptible de présenter des propriétés chélatantes et

iii) comparer la biodisponibilité obtenue à l'étape (i) et celle obtenue à l'étape (ii) moyennant quoi une biodisponibilité obtenue à l'étape (i) supérieure à celle obtenue à l'étape (ii) est une indication que la molécule testée à l'étape (i) présente des propriétés chélatantes vis-à-vis dudit radioélément.

15. Procédé selon la revendication 14, **caractérisé en ce que**, lors desdites étapes (i) et (ii), sont utilisées les mêmes conditions en termes de composition du gel mimant une zone de contamination, de nature et quantité de radioélément initialement présent dans ce gel, de composition de la solution mimant le fluide biologique, de technique de mesure du radioélément présent dans la solution et de temps t à laquelle cette mesure est réalisée, la seule différence étant la présence ou l'absence de la molécule à tester dans la solution mimant le fluide biologique.

**Patentansprüche**

1. Verfahren zum Vorhersagen der Bioverfügbarkeit eines Radioelements in einem lebenden Tierorganismus nach Kontamination dieses Organismus durch das Radioelement, umfassend die Schritte:

   a) Herstellen eines Gels, in dem das Radioelement gleichmäßig verteilt ist, wobei das Gel den Kontaminationsbereich in dem lebenden Tierorganismus nachahmt;

   b) Inkontaktbringen des in Schritt (a) hergestellten Gels mit einer Lösung, die eine biologische Flüssigkeit nachahmt, die dem Kontaminationsbereich in dem lebenden Tierorganismus zugeordnet ist, dann Verrühren des Ganzen, und

   c) Messen der Menge des Radioelements in der Lösung zu einem Zeitpunkt t, wobei die Messung es ermöglicht, die Bioverfügbarkeit des Radioelements in dem lebenden Tierorganismus vorherzusagen.

2. Verfahren nach Anspruch 1, **dadurch gekennzeichnet, dass** das radioaktive Element ein Aktinid ist.

3. Verfahren nach Anspruch 1 oder 2, **dadurch gekennzeichnet, dass** das Gel in dem Schritt (a) ausgehend von zumindest einer Verbindung mit kolloidalen Eigenschaften, einer Lösung, Gellösung genannt, und zumindest einem Radioelement hergestellt wird.

4. Verfahren nach Anspruch 3, **dadurch gekennzeichnet, dass** die Verbindung mit kolloidalen Eigenschaften ein Polysaccharid, insbesondere ausgewählt aus der Gruppe bestehend aus Agarose, Sucrose, Sepharose, Chitosan, Xanthan, Carrageenan, Dextran, Agar, Alginat oder deren Gemische, ist.

5. Verfahren nach Anspruch 3 oder 4, **dadurch gekennzeichnet, dass** das Verhältnis der Verbindung(en) mit kolloidalen Eigenschaften (Gewicht ausgedrückt in g) zur Gellösung (Volumen ausgedrückt in ml) zwischen 1 und 5%, typischerweise zwischen 2 und 4%, liegt.

6. Verfahren nach einem der Ansprüche 3 bis 5, **dadurch gekennzeichnet, dass** die Gellösung eine wässrige Lösung ist, wie beispielsweise ein physiologisches Serum, gegebenenfalls ergänzt mit einem oder mehreren organischen oder anorganischen Salzen, ausgewählt aus der Gruppe bestehend aus Natriumchlorid (NaCl), Kaliumchlorid (KCl), Natriumbikarbonat ($NaHCO_3$), Natriumphosphat ($Na_3PO_4$), Natriumhydrogenphosphat ($Na_2HPO_4$), Natriumdihydrogenphosphat ($NaH_2PO_4$), Kaliumhydrogenphosphat ($K_2HPO_4$), Kaliumdihydrogenphosphat ($KH_2PO_4$), Salzsäure (HCl), Magnesiumchlorid ($MgCl_2$), Calciumchlorid ($CaCl_2$), Natriumsulfat ($Na_2SO_4$), Natriumhydroxid (NaOH), Kaliumhydroxid (KOH), Natriumacetat ($CH_3COONa$), Natriumtartrat ($Na_2C_4H_4O_6$), Natriumlaktat ($C(OH)(CH_3)COONa$), Natriumpyruvat ($CH_3C(O)COONa$), Natriumcitrat ($Na_3C_6H_5O_7$) und Zitronensäure ($C_6H_8O_7$).

7. Verfahren nach einem der vorangehenden Ansprüche, **dadurch gekennzeichnet, dass** das Gel eine oder mehrere Verbindungen enthält, ausgewählt aus der Gruppe bestehend aus Pepsin, Lecithin, Glycin, Glucose, Lysozym, Albumin, Natrium-Taurocholat, Maleinsäure, Transferrin, Ferritin, Fibrin, Hyaluronsäure, Glucosamin, Fibronektin, Lamininin, einem Mucin, Keratinen, Kollagenen, Chondroitin, Osteopontin, Hydroxyapatit und Glutaminsäure.

8. Verfahren nach einem der Ansprüche 3 bis 7, **dadurch gekennzeichnet, dass** in Schritt (a) das Radioelement als Suspension oder Lösung der Gellösung zugegeben wird, die zumindest eine Verbindung mit kolloidalen Eigenschaften enthält.

9. Verfahren nach einem der vorangehenden Ansprüche, **dadurch gekennzeichnet, dass** die Lösung aus Schritt (b) eine wässrige Lösung ist, die ein oder mehrere organische oder anorganische Salze enthält, ausgewählt aus der Gruppe bestehend aus Natriumchlorid (NaCl), Kaliumchlorid (KCl), Natriumbikarbonat (NaHCO$_3$), Natriumphosphat (Na$_3$PO$_4$), Natriumhydrogenphosphat (Na$_2$HPO$_4$), Natriumdihydrogenphosphat (NaH$_2$PO$_4$), Kaliumhydrogenphosphat (K$_2$HPO$_4$), Kaliumdihydrogenphosphat (KH$_2$PO$_4$), Salzsäure (HCl), Magnesiumchlorid (MgCl$_2$), Calciumchlorid (CaCl$_2$), Natriumsulfat (Na$_2$SO$_4$), Natriumhydroxid (NaOH), Kaliumhydroxid (KOH), Natriumacetat (CH$_3$COONa), Natriumtartrat (Na$_2$C$_4$H$_4$O$_6$), Natriumlaktat (C(OH)(CH$_3$)COONa), Natriumpyruvat (CH$_3$C(O)COONa), Natriumcitrat (Na$_3$C$_6$H$_5$O$_7$) und Zitronensäure (C$_6$H$_8$O$_7$).

10. Verfahren nach einem der Ansprüche 3 bis 9, **dadurch gekennzeichnet, dass** die Gellösung und die in Schritt (b) hergestellte Lösung ein oder mehrere Salze, identisch oder verschieden, enthalten.

11. Verfahren nach einem der vorangehenden Ansprüche, **dadurch gekennzeichnet, dass** die Lösung aus Schritt (b) ferner eine oder mehrere Verbindungen enthält, ausgewählt aus der Gruppe bestehend aus Pepsin, Lecithin, Glycin, Glucose, Lysozym, Albumin, Natrium-Taurocholat, Maleinsäure, Transferrin, Ferritin und Fibrin.

12. Verfahren nach einem der vorangehenden Ansprüche, **dadurch gekennzeichnet, dass** die Lösung aus Schritt (b) in einem Rührwerk-Inkubator hergestellt wird, der eine Temperatur in der Größenordnung von 37 °C aufweist und dessen kontrollierte Atmosphärenzusammensetzung feuchte Luft mit 5% CO$_2$ ist.

13. Verwendung eines Verfahrens nach einem der Ansprüche 1 bis 12 zum Identifizieren eines Moleküls mit chelatbildenden Eigenschaften in Bezug auf ein vorgegebenes Radioelement und/oder zum Charakterisieren der chelatbildenden Eigenschaften eines Moleküls.

14. Verfahren zum Identifizieren eines Moleküls mit chelatbildenden Eigenschaften in Bezug auf ein Radioelement, umfassend die Schritte:

i) Messen der Bioverfügbarkeit des Radioelements mit dem Verfahren nach einem der Ansprüche 1 bis 12, wobei die das biologische Fluid nachahmende Lösung ein Molekül enthält, das chelatbildende Eigenschaften aufweisen kann,

ii) Messen der Bioverfügbarkeit des Radioelements mit dem Verfahren nach einem der Ansprüche 1 bis 12 unter den gleichen Bedingungen wie in Schritt (i), wobei die das biologische Fluid nachahmende Lösung kein Molekül enthält, das chelatbildende Eigenschaften aufweisen kann, und

iii) Vergleichen der in Schritt (i) erhaltenen Bioverfügbarkeit mit der in Schritt (ii) erhaltenen, wodurch eine in Schritt (i) erhaltene Bioverfügbarkeit, die höher ist als die in Schritt (ii) erhaltene, ein Hinweis dafür ist, dass das in Schritt (i) getestete Molekül chelatbildende Eigenschaften in Bezug auf das Radioelement aufweist.

15. Verfahren nach Anspruch 14, **dadurch gekennzeichnet, dass** bei den Schritten (i) und (ii) die gleichen Bedingungen bezüglich der Zusammensetzung des einen Kontaminationsbereich nachahmenden Gels, der Art und Menge des ursprünglich in diesem Gel vorhandenen Radioelements, der Zusammensetzung der das biologische Fluid nachahmenden Lösung, der Messtechnik für das in der Lösung vorhandene Radioelement und bezüglich der Zeit t, zu der diese Messung durchgeführt wird, Anwendung finden, wobei der einzige Unterschied im Vorhandensein bzw. Nichtvorhandensein des zu testenden Moleküls in der das biologische Fluid nachahmenden Lösung liegt.

## Claims

1. Method for predicting the bioavailability of a radioelement in a living animal organism further to contamination of this organism by said radioelement, comprising the steps of:

a) preparing a gel in which said radioelement is uniformly distributed, said gel mimicking the contamination site in said living animal organism;
b) placing the gel prepared at said step (a) in contact with a solution mimicking a biological fluid associated with the contamination site in said living animal organism, then placing the whole under agitation; and
c) measuring, at a time t, the amount of said radioelement in said solution, said measurement allowing prediction of the bioavailability of said radioelement in said living animal organism.

2. The method according to claim 1, **characterized in that** said radioelement is an actinide.

3. The method according to claim 1 or 2, **characterized in that** the gel is prepared at step said (a) from at least one compound having colloidal properties, a solution called gel solution and at least one radioelement.

4. The method according to claim 3, **characterized in that** said compound having colloidal properties is a polysaccharide in particular selected from the group consisting of agarose, sucrose, sepharose, chitosan, xanthan, carrageenan, dextran, agar, alginate or mixtures thereof.

5. The method according to claim 3 or 4, **characterized in that** the ratio between compound(s) having colloidal properties (weight expressed in g)/ gel solution (volume expressed in ml) is between 1 and 5 %, typically between 2 and 4 %.

6. The method according to any of claims 3 to 5, **characterized in that** said gel solution is an aqueous solution e.g. physiological saline solution optionally completed with one or more organic or inorganic salts selected from the group consisting of sodium chloride (NaCl), potassium chloride (KCl), sodium bicarbonate (NaHCO$_3$), sodium phosphate (Na$_3$PO$_4$), sodium hydrogen phosphate (Na$_2$HPO$_4$), sodium dihydrogen phosphate (NaH$_2$PO$_4$), potassium hydrogen phosphate (K$_2$HPO$_4$), potassium dihydrogen phosphate (KH$_2$PO$_4$), hydrochloric acid (HCl), magnesium chloride (MgCl$_2$), calcium chloride (CaCl$_2$), sodium sulfate (Na$_2$SO$_4$), sodium hydroxide (NaOH), potassium hydroxide (KOH), sodium acetate (CH$_3$COONa), sodium tartrate (Na$_2$C$_4$H$_4$O$_6$), sodium lactate (C(OH)(CH$_3$)COONa), sodium pyruvate (CH$_3$C(O)COONa), sodium citrate (Na$_3$C$_6$H$_5$O$_7$) and citric acid (C$_6$H$_8$O$_7$).

7. The method according to any of the preceding claims, **characterized in that** the gel comprises one or more compounds selected from the group consisting of pepsin, lecithin, glycine, glucose, lysozyme, albumin, sodium taurocholate, maleic acid, transferrin, ferritin, fibrin, hyaluronic acid, glucosamine, fibronectin, laminin, mucin, keratins, collagens, chondroitin, osteopontin, hydroxyapatite and glutamic acid.

8. The method according to any of claims 3 to 7 **characterized in that**, at step (a), the radioelement is added in the form of a suspension or of a solution to the gel solution containing at least one compound having colloidal properties.

9. The method according to any of the preceding claims, **characterized in that** the solution of said step (b) is an aqueous solution comprising one or more organic or inorganic salts selected from the group consisting of sodium chloride (NaCl), potassium chloride (KCl), sodium bicarbonate (NaHCO$_3$), sodium phosphate (Na$_3$PO$_4$), sodium hydrogen phosphate (Na$_2$HPO$_4$), sodium dihydrogen phosphate (NaH$_2$PO$_4$), potassium hydrogen phosphate (K$_2$HPO$_4$), potassium dihydrogen phosphate (KH$_2$PO$_4$), hydrochloric acid (HCl), magnesium chloride (MgCl$_2$), calcium chloride (CaCl$_2$), sodium sulfate (Na$_2$SO$_4$), sodium hydroxide (NaOH), potassium hydroxide (KOH), sodium acetate (CH$_3$COONa), sodium tartrate (Na$_2$C$_4$H$_4$O$_6$), sodium lactate (C(OH)(CH$_3$)COONa), sodium pyruvate (CH$_3$C(O)COONa), sodium citrate (Na$_3$C$_6$H$_5$O$_7$) and citric acid (C$_6$H$_8$O$_7$).

10. The method according to any of claims 3 to 9, **characterized in that** said gel solution and said solution used at step (b) comprise one or more salts, the same or different.

11. The method according to any of the preceding claims, **characterized in that** the solution of said step (b) further comprises one or more compounds selected from the group consisting of pepsin, lecithin, glycine, glucose, lysozyme, albumin, sodium taurocholate, maleic acid, transferrin, ferritin and fibrin.

12. The method according to any of the preceding claims, **characterized in that** the solution of said step (b) is prepared in an incubator with agitation having a temperature of approximately 37°C, the controlled composition of the atmosphere being humid air comprising 5 % CO$_2$.

13. Use of a method according to any of claims 1 to 12, to identify a molecule having chelating properties towards a given radioelement and/or to characterize the chelating properties of a molecule.

14. Method for identifying a molecule having chelating properties towards a radioelement, comprising the steps of:

i) measuring the bioavailability of said radioelement with the method according to any of claims 1 to 12, wherein the solution mimicking the biological fluid comprises a molecule likely to exhibit chelating properties;
ii) measuring the bioavailability of said radioelement with the method according to any of claims 1 to 12 under the same conditions as at step (i), the solution mimicking the biological fluid being free of said molecule likely to exhibit chelating properties; and

iii) comparing the bioavailability obtained at step (i) with that obtained at step (ii) whereby, if the bioavailability obtained at step (i) is higher than obtained at step (ii), it is an indication that the molecule tested at step (i) has chelating properties towards said radioelement.

15. The method according to claim 14, **characterized in that** at said steps (i) and (ii), the same conditions are used in terms of composition of the gel mimicking a contamination site, type and amount of radioelement initially contained in this gel, composition of the solution mimicking the biological fluid, technique for measuring the radioelement contained in the solution, and time t at which this measurement is performed, the only difference being the presence or absence of the molecule to be tested in the solution mimicking the biological fluid.

FIG.1

FIG.2

FIG.3

FIG.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **H. S. SARKAR et al.** *Nucl. Med. Biol.,* 1995
- **ANSOBORLO et al.** Review and critical analysis of available in vitro dissolution tests. *Health Physics,* 1999, vol. 77, 638-645
- **DAVISON ; ZHANG.** In situ speciation measurements of trace components in natural waters using thin film gels. *Nature,* 1994, vol. 367, 546-548
- **KLOSE et al.** Towards more realistic in vitro release measurement techniques for biodegradable microparticles. *Pharmaceutical Reseach,* 2009, vol. 26, 691-699

- **HOANG THI et al.** Bone implants modified with cyclodextrin: Study of drug release in bulk. *International Journal of Pharmaceutics,* 2010, vol. 400, 74-85
- **CHAIBVA ; WALKER.** The comparison of in vitro release methods for the evaluation of oxytocin release from pluronic® F127 parenteral formulations. *Dissolution Technologies,* 2007, vol. 14, 15-25
- **MARQUES et al.** Simulated biological fluids with possible application in dissolution testing. *Dissolution Technologies,* 2011, vol. 18, 15-28
- **ZAMMIT et al.** Effects on fluid and Na+ flux of varying luminal hydraulic restance in rat colon in vivo. *Journal of Physiology,* 1994, vol. 477, 539-548